(19) Europäisches Patentamt
European Patent Office
Office européen des brevets

(11) EP 0 623 577 B1

(12) **EUROPÄISCHE PATENTSCHRIFT**

(45) Veröffentlichungstag und Bekanntmachung des Hinweises auf die Patenterteilung:
**15.04.1998 Patentblatt 1998/16**

(51) Int. Cl.[6]: **C07C 51/60**, C07C 67/14

(21) Anmeldenummer: **94106032.9**

(22) Anmeldetag: **19.04.1994**

# (54) Herstellung von Carbonsäurehalogeniden und Carboxylat-Salzen

Preparation of halogenides and salts of carboxylic acids

Préparation d'halogénures et de sels d'acides carboxyliques

(84) Benannte Vertragsstaaten:
**AT BE CH DE DK ES FR GB GR IE IT LI LU NL PT**

(30) Priorität: **27.04.1993 DE 4313793**

(43) Veröffentlichungstag der Anmeldung:
**09.11.1994 Patentblatt 1994/45**

(73) Patentinhaber:
**Solvay Fluor und Derivate GmbH**
**D-30173 Hannover (DE)**

(72) Erfinder:
- **Braun, Max, Dr.**
  **D-30900 Wedemark (DE)**
- **Rudolph, Werner, Dr.**
  **D-30559 Hannover (DE)**
- **Palsherm, Stefan**
  **D-30890 Barsinghausen (DE)**
- **Eichholz, Kerstin**
  **D-30855 Langenhagen (DE)**

(74) Vertreter: **Lauer, Dieter, Dr.**
**Solvay Pharmaceuticals GmbH,**
**Hans-Böckler-Allee 20**
**30173 Hannover (DE)**

(56) Entgegenhaltungen:
**EP-A- 0 002 989** **EP-A- 0 209 157**
**CH-A- 313 549**

- **PATENT ABSTRACTS OF JAPAN vol. 6, no. 68 (C-100)(946) 30. April 1982 & JP-A-57 007 438 (NIPPON SENBAI KOSHA ET AL.) 14. Januar 1982**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 623 577 B1

## Beschreibung

Die Erfindung betrifft ein Verfahren zur Herstellung von Carbonsäurehalogeniden und Carboxylat-Salzen.

Carbonsäurehalogenide, beispielsweise Trifluoracetylchlorid, Trifluoracetylbromid oder Trifluoracetyljodid, sind wertvolle Zwischenprodukte in der chemischen Synthese, beispielsweise bei der Herstellung von Herbiziden, Surfactants und Pharmazeutika. Trifluoracetylchlorid beispielsweise ist ein Polymerisationsinitiator für Tetrafluorethylen.

Auch Carbonsäuresalze sind wertvolle Zwischenprodukte in der Synthese. So kann man halogensubstituierte aromatische Verbindungen mit Natriumtrifluoracetat unter Natriumchlorid-Abspaltung und Decarboxylierung zu der entsprechenden trifluormethylierten aromatischen Verbindung umsetzen.

Ein technisches Verfahren zur Herstellung von Trifluoressigsäurechlorid wird in der WO 81/01406 beschrieben. Dabei wird 1.1.1.-Trifluor-2.2.2.-trichlorethan mit Schwefeltrioxid in Gegenwart von Quecksilbersalzen und zusätzlich Borhalogenid und/oder Halogensulfonsäure umgesetzt. Das gebildete $CF_3C(O)Cl$ kann z. B. mit Alkoholen zu Estern der Trifluoressigsäure umgesetzt werden. Carbonsäurebromide und Carbonsäureiodide können aus den jeweiligen Carbonsäurechloriden mit wasserfreiem Bromwasserstoff oder Jodwasserstoff hergestellt werden, siehe R.N. Haszeldine, J. Chem. Soc. 1951, Seiten 584 bis 587. Ein anderes technisches Verfahren zur Herstellung von Trifluoracetylbromid und Trifluoracetyljodid offenbart die japanische Anmeldung JP-A 2/262 530. Bei diesem Verfahren wird das entsprechende Acetylfluorid mit Lithiumbromid oder Lithiumjodid zur Reaktion gebracht. Weiterhin kennt man die Herstellung von Trifluoracetylhalogeniden durch Umsetzung von Trifluoressigsäureanhydrid mit Phosphorsäurechloriden oder Phosphinsäurechloriden. Dabei entsteht als Nebenprodukt ein gemischtes Anhydrid aus Phosphonsäure oder Phosphinsäure und Trifluoressigsäure, das in der Technik wertlos ist. Dieses Verfahren wird von J. Helinski et al. in Phosphorus Sulfur Silicon Relat. Chem. 54 (1990), Seiten 225 und 226 beschrieben.

Die CH-A-313 549 offenbart ein Verfahren zur Herstellung von Carbonsäurehalogeniden. Dabei werden die leicht zugänglichen Alkali- und Erdalkalihalogenide als Halogenlieferanten mit Carbonsäureanhydriden, insbesondere Essigsäureanhydrid, in Gegenwart von Katalysatoren wie Bortrifluorid oder Aluminiumchlorid umgesetzt.

Die bekannten Verfahren insbesondere zur Herstellung von Carbonsäurehalogeniden sind technisch schwierig durchzuführen, oder es bilden sich unerwünschte Abfallprodukte. Aufgabe der vorliegenden Erfindung ist es, ein technisch einfach durchführbares Verfahren zur Herstellung insbesondere von Carbonsäurehalogeniden anzugeben, das insbesondere die Herstellung von Carbonsäurehalogeniden ohne Abfallprodukte gewährleistet. Diese Aufgabe wird durch das erfindungsgemäße Verfahren zur simultanen Herstellung von Carbonsäurehalogeniden und Carboxylat-Salzen gelöst. Die erfindungsgemäße Simultanherstellung von Carbonsäurehalogeniden und Carboxylat-Salzen, gebildet aus Carboxylat-Anionen und 1/n Kationen $M^{n+}$, erfolgt durch Umsetzung von Verbindungen $M^{n+}(Hal^-)_n$, worin $M^{n+}$ ein "Onium"-Kation ist und $n^+$ die positive Ladung des Kations angibt, und worin $Hal^-$ Chlorid, Bromid oder Jodid bedeutet, mit einem Carbonsäureanhydrid oder durch Umsetzung von Verbindungen $M^{n+}(Hal^-)_n$, worin $M^{n+}$ das Kation eines Metalls der ersten, zweiten oder dritten Hauptgruppe oder ein "Onium"-Kation ist, $n^+$ die positive Ladung des Kations angibt und $Hal^-$ Chlorid oder Bromid bedeutet, mit einem Carbonsäureanhydrid einer Carbonsäure mit 2 bis 4 C-Atomen, die durch 1 bis 7 Halogenatome substituiert ist. Der Begriff "Carbonsäurehalogenid" bedeutet im Rahmen der vorliegenden Erfindung Carbonsäurechlorid, -bromid oder -iodid, vorzugsweise Carbonsäurechlorid.

Zweckmäßig setzt man etwa n Äquivalente des Carbonsäureanhydrids oder einen geringen Überschuß ein. Natürlich kann man, bei verringerter Ausbeute, das Carbonsäureanhydrid auch im Unterschuß einsetzen. Gute Ergebnisse erzielt man, wenn man 0,95 n bis 1,05 n Äquivalente des Carbonsäureanhydrids einsetzt. Beispielsweise kann man 1 mol $AlCl_3$(n = 3) mit 3 mol Trifluoressigsäureanhydrid zu Trifluoracetylchlorid und Aluminiumtrifluoracetat umsetzen.

Bevorzugte Metallkationen sind solche der 1., 2. und 3. Hauptgruppe, insbesondere Natrium, Kalium und Aluminium. Bei der vorliegenden Erfindung stellt "n" eine ganze Zahl dar, vorzugsweise 1, 2 oder 3.

Der Begriff "Onium"-Kation steht für Moleküle mit einem positiv geladenem Stickstoffatom.

Eine vorteilhafte Eigenschaft des erfindungsgemäßen Verfahrens ist es, daß ausschließlich Wertprodukte gebildet werden, ohne daß Abfallprodukte anfallen.

Gemäß einer bevorzugten Ausführungsform setzt man das Ausgangsmaterial $M^{n+}(Hal^-)_n$ in Form von wasserfreien Abfallprodukten ein, wie sie bei chemischen oder physikalischen Verfahren anfallen. "Onium"-halogenide dienen beispielsweise als Katalysatoren oder Phasentransferkatalysatoren, beispielsweise bei der Herstellung organischer Carbonate aus organischen Säuren, Kohlenmonoxid und Sauerstoff und in Anwesenheit weiterer katalytisch aktiver "Substanzen. "Onium"-halogenide fallen beispielsweise auch bei der basenkatalysierten Umsetzung von C-H-aciden Verbindungen, z.B. Malonsäureester, mit Carbonsäurehalogeniden an. Mit organischem Material verunreinigte Metallhalogenide, z.B. Natriumchlorid, sind Abfallprodukt bei der Umsetzung von Natriumsalzen von Carbonsäuren mit z.B. Halogenaromaten zu alkylierten Aromaten. Derartige Abfallprodukte oder verbrauchte Katalysatoren können nach dem erfindungsgemäßen Verfahren in Wertprodukte überführt werden.

Gemäß einer Variante des erfindungsgemäßen Verfahrens verzichtet man auf die Isolation des Carbonsäurehalogenids und des Carboxylat-Salzes. In dieser Variante wird das Carbonsäurehalogenid und Carboxylat-Salz enthaltende Reaktionsgemisch ohne Isolation weiterverarbeitet, beispielsweise zu Carbonsäureestern, s. u.

Gemäß einer anderen Variante des erfindungsgemäßen Verfahrens trennt man das Reaktionsgemisch in Carbonsäurehalogenid und Carboxylat-Salz auf. Dies ist sehr einfach möglich durch Abdampfen des flüchtigen Carbonsäurehalogenids, gewünschtenfalls im Vakuum. Die Reaktionsprodukte können dann der gewünschten Verwendung zugeführt werden.

Aus dem oben Gesagten ergibt sich bereits, daß man die bei der Umsetzung von Metall- oder "Onium"-halogenid mit Carbonsäureanhydriden erhaltenen Reaktionsprodukte bei Verfahren einsetzen kann, in denen wiederum Metall- oder "Onium"-halogenid freigesetzt wird, so beispielsweise in Acylierungsverfahren, Alkylierungsverfahren, bei der Veresterung oder bei der Ketonherstellung. Das dabei freigesetzte Halogenid kann nun wiederum mit Carbonsäureanhydrid umgesetzt und erneut regeneriert werden. Auf diese Weise ist eine Acylierung, Alkylierung, Veresterung oder Herstellung von Ketonen möglich, ohne daß Abfallsalze notwendig werden und ohne daß gegebenenfalls eine Hydrolyse erfolgen muß. Diese Ausführungsform des erfindungsgemäßen Verfahren ist besonders bevorzugt und dadurch gekennzeichnet, daß man das Carbonsäurehalogenid und/oder Carboxylat-Salz in Reaktionen verwendet, bei welchen erneut $M^{n+}(Hal^-)_n$ freigesetzt wird und dieses erneut mit Carbonsäureanhydrid umgesetzt und dabei regeneriert wird. Auf diese Weise ist eine kontinuierliche oder halbkontinuierliche Verfahrensweise ohne Anfall von Salzen möglich. Dabei ist zudem noch eine hydrolysefreie Arbeitsweise möglich.

Gegebenenfalls muß zur Erhöhung der Reaktionsgeschwindigkeit ein Lösungsmittel für das Anhydrid, beispielsweise ein Kohlenwasserstoff oder ein monomerer, oligomerer oder polymerer Ether oder ein Lösungsmittel für das Halogenid, beispielsweise eins der bekannten polaren Lösungsmittel wie Nitrile, Lactame, Ether etc. eingesetzt werden. Gegebenenfalls kann man die Reaktionsgeschwindigkeit auch durch Verwendung von Phasentransferkatalysatoren, wie Kronenethern erhöhen.

Sehr gute Ergebnisse werden erzielt, wenn man Carbonsäureanhydride von Carbonsäuren mit 2 bis 4 C-Atomen einsetzt. Besonders vorteilhaft setzt man Carbonsäureanhydride von Carbonsäuren mit 2 bis 4 C-Atomen ein, die durch 1 bis 7 Halogenatome, vorzugsweise 1 bis 7 Fluoratome, substituiert sind, wie beispielsweise Trifluoressigsäureanhydrid.

Als Halogenid setzt man vorzugsweise Natriumhalogenid oder Kaliumhalogenid oder ein "Onium"-halogenid des Stickstoffs ein. Halogenid steht vorzugsweise für Chlor, Brom und Jod, insbesondere für Chlor und Brom. Besonders gute Ergebnisse mit überraschend hohen Ausbeuten werden im erfindungsgemäßen Verfahren erzielt, wenn man ein "Onium"-Halogenid einsetzt. Somit steht $M^{n+}$ vorzugsweise für ein "Onium"-Kation des Stickstoffs der Formel $R^1 R^2 R^3 R^4 N^+$, worin $R^1$, $R^2$, $R^3$ und $R^4$ unabhängig voneinander Wasserstoff, Alkyl mit 1 bis 20 C-Atomen, Aryl oder Aralkyl stehen, oder worin $R^1$ und $R^2$, oder worin $R^3$ und $R^4$, oder worin $R^1$, $R^2$ und $R^3$ oder worin $R^1$, $R^2$, $R^3$ und $R^4$, gegebenenfalls unter Einschluß des Stickstoff-Atoms, gesättigte oder ungesättigte Ringsysteme, insbesondere heteroaromatische Verbindungen, bilden. Aryl steht hier insbesondere für Phenyl und Phenyl, substituiert durch 1 oder mehrere Halogenatome und/oder substituiert durch 1 oder mehrere C1-C2-Alkylgruppen. Sehr gut geeignet sind Salze, in denen $M^{n+}$ für Ammonium, Piperidinium, Pyridinium oder $R^{1'} R^{2'} R^{3'} R^{4'} N^+$ steht, worin $R^{1'}$, $R^{2'}$, $R^{3'}$ und $R^{4'}$ unabhängig voneinander für Wasserstoff, Alkyl mit 1 bis 15 C-Atomen oder Benzyl stehen. Als gute Beispiele seien genannt Pyridiniumhydrohalogenide, Aniliniumhydrohalogenide, Piperidiniumhydrohalogenide, Benzyltriethylammoniumhydrohalogenide und Triethylammoniumhydrohalogenide, vorzugsweise die Chloride und Bromide, insbesondere die Chloride.

Eine besonders bevorzugte Variante der Erfindung sieht vor, daß man das Gemisch von Carbonsäurehalogenid und Carboxylat-Salz während oder nach seiner Herstellung mit einem Alkohol unter Bildung eines Carbonsäureesters umsetzt. Viele Ester von Carbonsäuren werden in der Technik als solche verwendet. Essigester und andere Carbonsäureester dienen beispielsweise als Lösungs- oder Reinigungsmittel, andere Ester, z. B. von Bernsteinsäure, werden zur Aromatisierung eingesetzt. Der Trifluoressigsäureethylester ist beispielsweise ein Lösungsmittel für die Chlorierung von Paraffinen oder die Polymerisation von Olefinoxiden. Viele Carbonsäureester sind auch Zwischenprodukte in der chemischen Synthese. Der Trifluoressigsäuremethylester und Trifluoressigsäure-1.1.1-Trifluorethylester liefern nach Hydrierung Trifluorethanol. Trifluorethanol wird als Lösungsmittel und als Zwischenprodukt, beispielsweise bei der Herstellung des Lösungsmittels und Anästhetikums Isoflurane, verwendet. Ester der Trifluoressigsäure dienen auch zur Einführung bzw. zur Herstellung von biologisch wirksamen Verbindungen, die eine $CF_3$-Gruppe aufweisen. Beispielsweise kann man durch N-Acylierung mit Trifluoressigsäuremethylester Peptide mit hormonaler Aktivität herstellen. Der Trifluorethylester liefert mit Kampfer-Derivaten Shiftreagenzien für die NMR-Analyse. Der Trifluormethylphenylester liefert nach Fries'scher Verschiebung mit Aluminiumchlorid das entsprechende trifluoracetylierte Phenol, welches ein Synthesebaustein für Pharmazeutika ist. Viele weitere Anwendungszwecke von Estern sind dem Fachmann bekannt, beispielsweise die Verwendung bei der Herstellung von Pharmazeutika, Photosensibilisatoren und Farbstoffen.

Die Herstellung von Carbonsäureestern erfolgt üblicherweise durch Umsetzung der entsprechenden Alkohole mit den Carbonsäuren unter saurer Katalyse. Dabei muß das entstehende Reaktionswasser zur Gleichgewichtsverschiebung entfernt werden; bei fluorierten Derivaten kann dies aufgrund der bevorzugten Bindung von Wasser (an den Carbonylfunktionen als Hydrate) zu Schwierigkeiten führen. Es ist auch bereits bekannt, daß man Carbonsäureester aus Carbonsäurechloriden und Alkoholen unter Basenkatalyse herstellen kann. Dabei ist jedoch eine hydrolytische Aufarbeitung notwendig, außerdem entstehen Abfallsalze, beispielsweise Pyridinhydrochlorid, welche entsorgt werden müssen. Die Umsetzung von Carbonsäurehalogeniden mit Alkoholen ohne Basenkatalyse verläuft mit geringer Reaktionsgeschwindigkeit.

Der Erfindung liegt die Erkenntnis zugrunde, daß das erhaltene Produktgemisch, welches Carbonsäurehalogenid und Carboxylat-Salz umfaßt, mit Alkoholen Carbonsäureester liefert. Dabei übt das Carboxylat-Salz überraschenderweise ein katalytische Wirkung aus.

Natürlich kann man Carbonsäurehalogenid und Carboxylat-Salz zunächst getrennt isolieren und dann in gewünschten Mengen mit Alkoholen umsetzen.

Ein Zusatz von Säure, z. B. einer Carbonsäure, ist zwar möglich, aber nicht notwendig und erfolgt vorzugsweise, insbesondere bei in-situ-Veresterung, nicht.

Bevorzugt geht man zwecks Esterherstellung von Carbonsäurechloriden aus. Als Katalysator verwendet man vorzugsweise ein "Onium"-Salz der Carbonsäure.

Die Variante des erfindungsgemäßen Verfahrens kann prinzipiell für die Herstellung beliebiger Ester von beliebigen Carbonsäuren mit beliebigen Alkoholen angewendet werden. Eine bevorzugte Ausführungsform der Variante sieht vor, daß man ein Carbonsäurechlorid der Formel $R^aC(O)Cl$ (I) einsetzt, worin $R^a$ für Alkyl mit 1 bis 6 C-Atomen; durch mindestens 1 Halogenatom substituiertes Alkyl mit 1 bis 6 C-Atomen; Phenyl, Tolyl; durch mindestens 1 Halogenatom substituiertes Phenyl oder Tolyl steht.

Weiterhin ist es bevorzugt, daß man einen Alkohol der Formel $R^bOH$ (II) einsetzt, worin $R^b$ für Alkyl oder Alkenyl mit 1 bis 8 C-Atomen; durch mindestens 1 Halogenatom substituiertes Alkyl oder Alkenyl mit 1 bis 8 C-Atomen; Phenyl, Tolyl; Benzyl; durch mindestens 1 Halogenatom und/oder mindestens eine Nitrogruppe substituiertes Phenyl, Tolyl oder Benzyl steht.

Ganz besonders bevorzugt ist es, daß $R^a$ für durch mindestens 1 Fluoratom substituiertes Alkyl mit 1 bis 4 C-Atomen und $R^b$ für Alkyl oder Alkenyl mit 1 bis 4 C-Atomen; durch mindestens 1 Halogenatom substituiertes Alkyl oder Alkenyl mit 1 bis 4 C-Atomen; Phenyl; durch mindestens 1 Halogenatom und/oder durch mindestens eine Nitrogruppe substituiertes Phenyl steht. Insbesondere bedeutet Perfluormethyl, Perfluorethyl oder Perfluorpropyl. Insbesondere bevorzugt steht $R^b$ für Alkyl oder Alkenyl mit 1 bis 3 C-Atomen; durch mindestens 1 Fluoratom substituiertes Alkyl oder Alkenyl mit 1 bis 3 C-Atomen; Phenyl; durch mindestens 1 Fluoratom und/oder mindestens eine Nitrogruppe substituiertes Phenyl.

Besonders gut geeignet ist die Verfahrensvariante zur Herstellung von Estern der durch ein oder mehrere Fluoratome substituierten Essigsäure. Beispielsweise kann man Trifluoressigsäurephenylester nach dem erfindungsgemäßen Verfahren herstellen. Besonders gut geeignet ist das erfindungsgemäße Verfahren zur Herstellung von Estern der Trifluoressigsäure mit 1.1.1-Trifluorethanol, Pentafluorpropanol, Methanol, Ethanol, Isopropanol, 4-Nitro-Phenol, Pentafluorphenol und Allylalkohol.

Das Molverhältnis zwischen Carbonsäurehalogenid und Alkohol liegt vorteilhafterweise oberhalb von 0,9. Der Alkohol kann auch in höherem Überschuß eingesetzt werden und dient als Lösungsmittel, besonders wenn es sich um einen durch elektronenziehende Gruppen, beispielsweise Fluor-Atome, substituierten Alkohol handelt. Zweckmäßig liegt das Molverhältnis zwischen Alkohol und Carbonsäurehalogenid zwischen 0,9:1 und 5:1.

Die Temperatur, bei der die Umsetzung durchgeführt wird, liegt bei Umgebungstemperatur (etwa 20 °C) bis hin zum Siedepunkt der Mischung, beispielsweise bis hin zu 100 °C. Man arbeitet bei Umgebungsdruck (etwa 1 bar abs.) oder gewünschtenfalls auch bei erhöhtem Druck, beispielsweise bei bis zu 5 bar abs.

Das Alkalimetall- oder "Onium"-Salz kann in katalytischen oder molaren Mengen anwesend sein. Zweckmäßig liegt das Mol-Verhältnis zwischen Säurehalogenid und dem Carbonsäuresalz im Be reich von 1:1 bis 20.000:1.

Gemäß einer besonderen Ausführungsform der Erfindung erzeugt man das Säurechlorid bzw. das Säurebromid und das Alkalimetall- oder "Onium"-Salz der Carbonsäure in situ. Hierzu setzt man das entsprechende Alkalimetall- oder "Onium"-Halogenid, vorzugsweise das Chlorid oder Bromid, insbesonder das Chlorid, mit dem Anhydrid der einzusetzenden Carbonsäure um. Bei dieser Umsetzung bildet sich aus dem Anhydrid der Carbonsäure das entsprechende Säurehalogenid und das entsprechende Salz. Bei dieser Ausführungsform kann man als Alkalimetall- oder "Onium"-Halogenid verbrauchte Halogenid-Katalysatoren einsetzen, die auf diese Weise in Wertprodukte überführt werden können.

Das erfindungsgemäße Verfahren weist eine Vielzahl von Vorteilen auf. So läuft es beispielsweise bereits bei Umgebungstemperatur ab, obwohl man natürlich auf höhere Temperatur, beispielsweise auf bis zu 60 °C oder höher erhitzen kann. Weiterhin kann man mit dem erfindungsgemäßen Verfahren spezielle Säurehalogenide erzeugen, die mit anderen Verfahren nur schwer zugänglich sind. Weiterhin kann man Salze, die bei technischen Verfahren als Abfallprodukte anfallen, in Wertprodukte überführen. Besonders vorteilhaft wendet man das erfindungsgemäße Verfahren dann an, wenn man Salze in Carbonsäurehalogenide und Carbonsäure-Salze überführt, die bei ihrer Weiterverarbeitung wiederum Salze erzeugen, die dann erneut nach dem erfindungsgemäßen Verfahren regeneriert werden können. Diese Vorgehensweise ermöglicht es, erstmals eine Vielzahl von Umsetzungen so durchzuführen, daß kein Salzabfall anfällt und/oder eine hydrolytische Aufarbeitung notwendig wird.

Die Variante der Erfindung mit Veresterung weist den Vorteil auf, daß man ohne hydrolytische Aufarbeitung in technisch einfacher Weise Carbonsäureester erzeugen kann. Bei den meisten Estern fallen auch keine Abfallstoffe wie Pyridinhydrochlorid an.

Die folgenden Beispiele sollen die Erfindung weiter erläutern, ohne sie in ihrem Umfang einzuschränken.

**Beispiel 1:**

Herstellung von Trifluoracetylchlorid und Pyridiniumtrifluoracetat durch Umsetzung von Pyridiniumhydrochlorid mit Trifluoressigsäureanhydrid.

$$Py \cdot HCl + [CF_3C(O)]_2O \rightarrow CF_3C(O)Cl + CF_3C(O)O \cdot PyH$$

Unter Feuchtigkeitsauschluß ($N_2$-Atmosphäre) wurden 26,17 g (0,226 mol) Pyridiniumhydrochlorid in 25 ml Trifluoressigsäure aufgenommen und 61,84 g (0,227 mol) Trifluoressigsäureanhydrid unter Rühren innerhalb einer Stunde kontinuierlich bei Raumtemperatur zugetropft. Sofort nach der Zugabe weniger Tropfen Trifluoressigsäureanhydrid war im Reaktionskolben eine starke Gasentwicklung zu erkennen. Das entstehende Trifluoracetylchlorid wurde in auf -78 °C gekühlte Kühlfallen einkondensiert. Nach beendeter Trifluoressigsäureanhydridzugabe wurde zum Austreiben von gelöstem Trifluoracetylchlorid die Reaktionslösung für eine Stunde auf 40 °C erwärmt. Die Ausbeute an einkondensiertem Trifluoracetylchlorid betrug 27,95 g (92,5 % der Theorie). Eine Chloridbestimmung ergab einen Restchlorgehalt in der Reaktionslösung von 626,2 mg Chlorid (17,66 mmol). Daraus ergibt sich eine Umsatzausbeute von > 99 %.

Der Reaktionsrückstand enthielt Pyridinium-trifluoracetat sowie etwas Trifluoressigsäure, welche durch Sprühtrocknung abgetrennt wurde. Zur Herstellung größerer Mengen wurde das Beispiel mehrfach wiederholt.

**Beispiel 2:**

Herstellung von Trifluoracetylchlorid und Piperidiniumtrifluoracetat durch Umsetzung von Piperidiniumhydrochlorid mit Trifluoressigsäureanhydrid.

$$Pip \cdot HCl + [CF_3C(O)]_2O \rightarrow CF_3C(O)Cl + CF_3C(O)O \cdot PipH$$

Unter Feuchtigkeitsausschluß ($N_2$-Atmosphäre) wurden 27,26 g (0,226 mol) Piperidiniumhydrochlorid in 25 ml Trifluoressigsäure aufgenommen und 61,84 g (0,227 mol) Trifluoressigsäureanhydrid unter Rühren innerhalb einer Stunde kontinuierlich bei Raumtemperatur zugetropft. Sofort nach der Zugabe weniger Tropfen Trifluoressigsäureanhydrid war im Reaktionskolben eine starke Gasentwicklung zu erkennen. Das entstehende Trifluoracetylchlorid wurd in auf -78 °C gekühlte Kühlfallen einkondensiert. Nach beendeter Trifluoressigsäureanhydridzugabe wurde zum Austreiben von gelöstem Trifluoracetylchlorid die Reaktionslösung für eine Stunde auf 40 °C erwärmt. Die Ausbeute an einkondensiertem Trifluoracetylchlorid betrug 26,97 g (89,9 % der Theorie). Eine Chloridbestimmung ergab einen Restchlorgehalt der Reaktionslösung von 778,8 mg Chlorid (21,97 mmol). Daraus ergibt sich eine Umsatzausbeute von 99,8 %.

**Beispiel 3:**

Herstellung von Trifluoracetylchlorid und Benzyltriethylammoniumtrifluoracetat durch Umsetzung von Benzyltriethylammoniumchlorid mit Trifluoressigsäureanhydrid.

$$(C_6H_5CH_2)Et_3N \cdot Cl + [CF_3C(O)]_2O \rightarrow CF_3C(O)Cl + CF_3C(O)O \cdot NEt_3(C_6H_5CH_2)$$

Unter Feuchtigkeitsausschluß ($N_2$-Atmosphäre) wurden 43,55 g (0,191 mol) Benzyltriethylammoniumhydrochlorid mit 21 ml Trifluoressigsäure aufgenommen und 52,20 g (0,249 mol) Trifluoressigsäureanhydrid unter Rühren innerhalb einer Stunde kontinuierlich bei Raumtemperatur zugetropft. Sofort nach der Zugabe weniger Tropfen Trifluoressigsäureanhydrid war im Reaktionskolben eine starke Gasentwicklung zu erkennen. Das entstehende Trifluoracetylchlorid wurde in auf -78 °C gekühlte Kühlfallen einkondensiert. Nach beendeter Trifluoressigsäureanhydridzugabe wurde zum Austreiben von gelöstem Trifluoracetylchlorid die Reaktionslösung für eine Stunde auf 40 °C erwärmt. Die Ausbeute an einkondensiertem Trifluoracetylchlorid betrug 20,26 g (80,1 % der Theorie). Eine Chloridbestimmung ergab einen Restchlorgehalt der Reaktionslösung von 15,3 mg (0,432 mmol) Chlorid. Daraus ergibt sich eine Umsatzausbeute von 87,1 %.

**Beispiel 4:**

Herstellung von Trifluoracetylchlorid und Triethylammoniumtrifluoracetat durch Umsetzung von Triethylammoniumhydrochlorid mit Trifluoressigsäureanhydrid.

$$Et_3N \cdot HCl + [CF_3C(O)]_2O \rightarrow CF_3C(O)Cl + CF_3C(O)O \cdot NHEt_3$$

Unter Feuchtigkeitsausschluß ($N_2$-Atmosphäre) wurden 31,11 g (0,226 mol) Triethylammoniumhydrochlorid mit 25 ml Trifluoressigsäure aufgenommen und 61,84 g (0,294 mol) Trifluoressigsäureanhydrid unter Rühren innerhalb einer Stunde kontinuierlich bei Raumtemperatur zugetropft. Sofort nach der Zugabe weniger Tropfen Trifluoressigsäureanhydrid war im Reaktionskolben eine starke Gasentwicklung zu erkennen. Das entstehende Trifluoracetylchlorid wurde in auf -78 °C gekühlte Kühlfallen einkondensiert. Nach beendeter Trifluoressigsäureanhydridzugabe wurde zum Austreiben von gelöstem Trifluoracetylchlorid die Reaktionslösung für eine Stunde auf 40 °C erwärmt. Die Ausbeute an einkondensiertem Trifluoracetylchlorid betrug 19,82 g (66,1 % der Theorie). Eine Chloridbestimmung ergab einen Restchlorgehalt der Reaktionslösung von 1,16 g Chlorid (32,7 mmol). Daraus ergibt sich eine Umsatzausbeute von 77,4 %).

**Beispiel 5:**

Herstellung von Trifluoracetylbromid und Pyridiniumtrifluoracetat durch Umsetzung von Pyridiniumhydrobromid mit Trifluoressigsäureanhydrid.

$$Py \cdot HBr + [CF_3C(O)]_2O \rightarrow CF_3C(O)Br + CF_3C(O)O \cdot PyH$$

Unter Feuchtigkeitsausschluß ($N_2$-Atmosphäre) wurden 36,16 g (0,226 mol) Pyridiniumhydrobromid mit 25 ml Trifluoressigsäure aufgenommen und 61,84 g (0,294 mol) Trifluoressigsäureanhydrid unter Rühren innerhalb einer Stunde kontinuierlich bei Raumtemperatur zugetropft. Sofort nach der Zugabe weniger Tropfen Trifluoressigsäureanhydrid war im Reaktionskolben eine starke Gasentwicklung zu erkennen. Das entstehende Trifluoracetylbromid wurde in auf -78 °C gekühlte Kühlfallen einkondensiert. Nach beendeter Trifluoressigsäureanhydridzugabe wurde zum Austreiben von gelöstem Trifluoracetylbromid die Reaktionslösung für eine Stunde auf 40 °C erwärmt. Die Ausbeute an einkondensiertem Trifluoracetylbromid betrug 28,18 g (70,5 % der Theorie) - Eine Bromidbestimmung ergab einen Restbromgehalt der Reaktionslösung von 4,90 g Bromid (61,3 mmol). Daraus ergibt sich eine Umsatzausbeute von 96,7 %.

**Beispiel 6:**

Herstellung von Trifluoracetylchlorid und Natriumtrifluoracetat durch Umsetzung von Natriumchlorid mit Trifluoressigsäureanhydrid in Gegenwart von Kronenether.

$$NaCl + [CF_3C(O)]_2O \rightarrow CF_3C(O)Cl + CF_3C(O)O \cdot Na$$

Unter Feuchtigkeitsausschluß wurden 13,25 g (0,226 mol) NaCl und 1,03 g (0,004 mol) 18-Krone-6 mit 25 ml Trifluoressigsäure aufgenommen und 32 ml (0,227 mol) Trifluoressigsäureanhydrid unter Rühren bei Raumtemperatur zugetropft. Das entstehende Trifluoracetylchlorid wurde in auf -78 °C gekühlte Kühlfallen einkondensiert. Nach beendeter Trifluoressigsäureanhydridzugabe wurde bei Raumtemperatur noch ca. 42 h gerührt. Die Ausbeute an in die Kühlfallen einkondensiertem Trifluoracetylchlorid betrug 28,29 g (55,90 % der Theorie). Eine Chloridbestimmung ergab einen Restchlorgehalt der Reaktionslösung von 2,95 g Chlorid (83,2 mmol). Daraus ergibt sich eine Umsatzausbeute von 63,3 %.

**Beispiel 7:**

Herstellung von Trifluoracetyljodid und Natriumtrifluoracetat durch Umsetzung von Natriumjodid mit Trifluoressigsäureanhydrid.

$$NaI + [CF_3C(O)]_2O \rightarrow CF_3C(O)I + CF_3C(O)O \cdot Na$$

Unter Feuchtigkeitsausschluß wurden 34,00 g (0,227 mol) Natriumjodid und 1,03 g (0,004 mol) 18-Krone-6 mit 25 ml Trifluoressigsäure aufgenommen und 32 ml (0,227 mol) Trifluoressigsäureanhydrid unter Rühren beim Raumtemperatur zugetropft. Sofort nach der Zugabe von Trifluoressigsäureanhydrid wurde die Lösung im Reaktionskolben fest. Deshalb wurden nochmals 25 ml Trifluoressigsäure zugegeben. Das entstehende Trifluoracetyljodid wurde in auf -78 °C gekühlte Kühlfallen einkondensiert. Nach beendeter Trifluoressigsäureanhydridzugabe wurde ca. 16 h bei Raumtemperatur gerührt. Am folgenden Tag wurde der Reaktionskolben ca. 8 h in ein Wasserbad mit 50 °C heißem Wasser gestellt, die Temperatur im Reaktionskolben stieg dabei auf 40 °C an. Die Ausbeute an Trifluoracetyljodid betrug 17,23 g (33,90 % der Theorie). Eine Jodidbestimmung ergab einen Restjodidgehalt der Reaktionslösung von 11,17 g Jodid (74,5 mmol). Daraus ergibt sich eine Umsatzausbeute von 50,5 %.

**Beispiel 8:**

Herstellung von Trifluoracetyljodid und Kaliumtrifluoracetat durch Umsetzung von Kaliumjodid mit Trifluoressigsäureanhydrid.

$$KI + [CF_3C(O)]_2O \rightarrow CF_3C(O)I + CF_3C(O)O \cdot K$$

Unter Feuchtigkeitsausschluß wurden 37,48 g (0,226 mol) Kaliumjodid und 0,99 g (0,004 mol) Kronenether mit 25 ml Trifluoressigsäure aufgenommen und 32 ml (0,227 mol) Trifluoressigsäureanhydrid unter Rühren bei Raumtemperatur zugetropft. Das entstehende Trifluoracetyljodid wurde in auf -78 °C gekühlte Kühlfallen einkondensiert. Nach beendeter Trifluoressigsäureanhydridzugabe wurde ca. 12 h bei Raumtemperatur gerührt. Am folgenden Tag wurde der Reaktionskolben ca. 10 h in ein Wasserbad mit 40 °C heißem Wasser gestellt. Die Ausbeute an Trifluoracetyljodid betrug 20,06 g (39,64 % der Theorie). Eine Jodidbestimmung ergab einen Restjodidgehalt der Reaktionslösung von 2,60 g Jodid (21,0 mmol). Daraus ergibt sich eine Umsatzausbeute von 43,9 %.

**Beispiel 9:**

Herstellung von Acetylchlorid und Pyridiniumacetat durch Umsetzung von Essigsäureanhydrid mit Pyridiniumchlorid.

$$Py \cdot HCl + [CH_3C(O)]_2O \rightarrow CH_3C(O)Cl + CH_3C(O)O \cdot PyH$$

Unter Feuchtigkeitsausschluß wurden in einer Claisen-Apparatur 45,33 g (0,392 mol) Pyridiniumhydrochlorid in 30 ml Essigsäure aufgenommen und 36 ml (0,381 mol) Essigsäureanhydrid unter Rühren bei Raumtemperatur vereinigt. Anschließend erwärmte man im Ölbad auf 100 °C und steigerte die Temperatur innerhalb von 7 h langsam bis auf 170 °C. Das entstandene Acetylchlorid wurde in einer auf 10 °C gekühlten Vorlage zusammen mit ebenfalls übergegangenem Anhydrid und Essigsäure aufgefangen. Die isolierte Ausbeute an Acetylchlorid in der Vorlage betrug nach dieser Reaktionszeit 7,1 % der Theorie. Der Umsatz betrug ca. 10 %.

In den Beispielen 6 bis 8 wurde 18-Krone-6 eingesetzt. Die in den Beispielen beschriebenen Reaktionen laufen auch ohne Zusatz von Kronenether ab, jedoch mit erheblich geringerer Reaktionsgeschwindigkeit.

**Beispiel 10:**

Herstellung von Trifluoracetylchlorid und Aluminiumtrifluoracetat durch Umsetzung von Aluminiumtrichlorid mit Trifluoressigsäureanhydrid.

$$AlCl_3 + 3\ (CF_3CO)_2O \rightarrow 3\ CF_3COCl + Al\ (OCOCF_3)_3$$

Unter Feuchtigkeitsausschluß ($N_2$-Atmosphäre) wurden 39,2 g (0,294 mol) $AlCl_3$ in 147 ml 1,4-Dioxan aufgenommen und an drei aufeinander folgenden Tagen mit insgesamt 185,25 g (0,882 mol) Trifluoressigsäureanhydrid (ein Äquivalent $(CF_3CO)_2O$ bezogen auf $AlCl_3$ pro Tag) unter Rühren innerhalb einer Stunde kontinuierlich bei Raumtemperatur zugetropft. Sofort nach der Zugabe weniger Tropfen Trifluoressigsäureanhydrid war im Reaktionskolben eine Gasentwicklung zu erkennen. Das entstehende Trifluoracetylchlorid wurde in auf -78 °C gekühlte Kühlfallen einkondensiert. Nach beendeter Trifluoressigsäureanhydridzugabe wurde zum Austreiben von gelöstem Trifluoracetylchlorid die Reaktionslösung für zwei Stunden auf 40 °C erwärmt. Die gesamte isolierte Ausbeute an einkondensiertem Trifluoracetylchlorid betrug über alle drei Tage 111,72 g, das entspricht 95,9 % der Theorie.

**Beispiel 11:**

Herstellung von Trifluoressigsäure-trifluorethylester durch Umsetzung von Trifluoracetylchlorid mit 2,2,2-Trifluorethanol in Gegenwart von Pyridiniumtrifluoracetat

$$CF_3COCl + CF_3CH_2OH \xrightarrow{PyTFA} CF_3COOCH_2CF_3 + HCl$$

In einer Labor-Umlaufapparatur (1 l Vierhalskolben mit KPG-Rührer als Vorlage, Prominent-Pumpe, 30 cm Kolonne mit Raschig-Ringen gefüllt) wurden 30 g (0,16 mol) des in Beispiel 1 erhaltenen Pyridiniumtrifluoracetats in 355 g (3,55 mol) 2,2,2-Trifluorethanol aufgenommen und bei einer Innentemperatur von 54 °C im Umlauf geführt. Anschließend wurden in den Kolben über ein Tauchrohr 134 g (1,01 mol) des analog zu Beispiel 1 erhaltenen Trifluoracetylchlorid innerhalb von 100 min zudosiert. Nach beendeter Trifluoracetylchloridzugabe ließ man 10 min nachreagieren. Anschließende fraktionierende Destillation des Reaktionsgemisches über eine Vigreuxkolonne (Übergangstemperatur: 54 bis 56 °C) ergab 177,8 g Trifluoressigsäure-trifluorethylester, dies entspricht einer Ausbeute von 89,8 % d. Theorie. Das im Vorlagekolben zurückgebliebene Trifluorethanol/Pyridiniumsalzgemisch kann erneut zur Veresterung mit gleicher Effektivität eingesetzt werden.

**Beispiele 12-17:**

Herstellung von Trifluoressigsäureestern durch Umsetzung von Trifluoracetylchlorid mit Methanol, Ethanol, Isopropanol, 4-Nitrophenol, Pentafluorphenol und Allylalkohol in Gegenwart von Pyridiniumtrifluoracetat

Analog der Vorschrift von Beispiel 11 wurden auch die entsprechenden Ester aus Trifluoracetylchlorid mit Methanol (94 %), Ethanol (96 %), Isopropanol (89 %), 4-Nitro-phenol (85 %), Pentafluorphenol (92 %) und Allylalkohol (81 %) dargestellt.

**Beispiel 18:**

Herstellung von Trifluoressigsäure-1.1.1-trifluorethylester in Gegenwart von Piperidiniumtrifluoracetat

Analog zu Beispiel 2 wurde, ausgehend von Trifluoressigsäureanhydrid und Piperidiniumchlorid, Trifluoracetylchlorid und Piperidiniumtrifluoracetat hergestellt und isoliert. Dann erfolgte analog zu Beispiel 11 die Herstellung von Trifluoressigsäure-1.1.1-trifluorethylester.

**Beispiel 19:**

Herstellung von Trifluoressigsäure-trifluorethylester durch Umsetzung von Trifluoressigsäureanhydrid mit 2,2,2-Trifluorethanol in Gegenwart von Pyridiniumhydrochlorid ("in situ"-Methode)

$$Py \cdot HCl + (CF_3CO)_2O + CF_3CO_2^- \cdot PyH + CF_3COCl \xrightarrow{CF_3CH_2OH} CF_3CO{-}OCH_2CF_3 + PyTFA + HCl$$

In einem 250 ml Dreihalskolben mit KPG-Rührer, Trockeneiskühler und Tropftrichter wurden 23,11 g (0,2 mol) Pyridiniumhydrochlorid und 20,0 g (0,2 mol) 2,2,2-Trifluorethanol vorgelegt. Anschließend tropfte man bei einer Reaktionsinnentemperatur von 52 bis 55 °C 42,01 g (0,2 mol) Trifluoressigsäureanhydrid innerhalb von 3 h zu. Die Ausbeute an Trifluoressigsäure-trifluorethylester betrug 98 % (GC).

**Patentansprüche**

1. Verfahren zur Simultanherstellung von Carbonsäurehalogeniden und Carboxylat-Salzen, gebildet aus Carboxylat-Anionen und 1/n Kationen $M^{n+}$, durch Umsetzung von Verbindungen $M^{n+}(Hal^{-})_n$, worin $M^{n+}$ ein "Onium"-Kation ist und $n^+$ die positive Ladung des Kations angibt, und worin $Hal^-$ Chlorid, Bromid oder Jodid bedeutet, mit einem Carbonsäureanhydrid oder durch Umsetzung von Verbindungen $M^{n+}(Hal^-)_n$, worin $M^{n+}$ das Kation eines Metalls der ersten, zweiten oder dritten Hauptgruppe oder ein "Onium"-Kation ist, $n^+$ die positive Ladung des Kations angibt und $Hal^-$ Chlorid oder Bromid bedeutet, mit einem Carbonsäureanhydrid einer Carbonsäure mit 2 bis 4 C-Atomen,

die durch 1 bis 7 Halogenatome substituiert ist.

2. Verfahren nach Anspruch 1, dadurch gekennzeichnet, daß man das $M^{n+}(Hal^{-})_n$ in Form von wasserfreien Abfallprodukten, z. B. verbrauchten Katalysatorzubereitungen, die $M^{n+}(Hal^{-})_n$ enthalten, einsetzt.

3. Verfahren nach Anspruch 1 oder 2, dadurch gekennzeichnet, daß man Carbonsäureanhydride von Carbonsäuren mit 2 bis 4 C-Atomen einsetzt.

4. Verfahren nach einem der vorhergehenden Ansprüche, dadurch gekennzeichnet, daß man Carbonsäureanhydride von Carbonsäuren mit 2 bis 4 C-Atomen einsetzt, die durch 1 bis 7 Halogenatome, vorzugsweise Fluortome, substituiert sind.

5. Verfahren nach einem der vorhergehenden Ansprüche, dadurch gekennzeichent, daß $M^{n+}$ für ein "Onium"-Kation des Stickstoffs steht.

6. Verfahren nach Anspruch 5, dadurch gekennzeichnet, daß $M^{n+}$ für ein "Onium"-Kation des Stickstoffs der Formel $R^1R^2R^3R^4N^+$ steht, worin $R^1$, $R^2$, $R^3$ und $R^4$ unabhängig voneinander Wasserstoff, Alkyl mit 1 bis 20 C-Atomen, Aryl oder Aralkyl stehen, oder worin $R^1$ und $R^2$, oder worin $R^3$ und $R^4$, oder worin $R^1$, $R^2$ und $R^3$ oder worin $R^1$, $R^2$, $R^3$ und $R^4$, gegebenenfalls unter Einschluß des Stickstoff-Atoms, gesättigte oder ungesättigte Ringsysteme bilden.

7. Verfahren nach Anspruch 6, dadurch gekennzeichnet, daß $M^{n+}$ für Ammonium, Piperidinium, Pyridinium oder $R^{1'}R^{2'}R^{3'}R^{4'}N^+$ steht, worin $R^{1'}$, $R^{2'}$, $R^{3'}$ und $R^{4'}$ unabhängig voneinander Wasserstoff, Alkyl mit 1 bis 15 C-Atomen oder Benzyl stehen.

8. Verfahren nach einem der vorhergehenden Ansprüche, dadurch gekennzeichnet, daß Halogenid für Chlorid steht.

9. Ausgestaltung des Verfahrens nach einem der vorhergehenden Ansprüche, dadurch gekennzeichnet, daß man das Carbonsäurehalogenid und Carboxylat-Salz enthaltende Reaktionsgemisch in Acylierungsverfahren einsetzt.

10. Verfahren nach einem der Ansprüche 1 bis 8, dadurch gekennzeichnet, daß man das Reaktionsgemisch in Carbonsäurehalogenid und Carboxylat-Salz trennt.

11. Ausgestaltung des Verfahrens nach einem der Ansprüche 1 bis 8 oder 10, dadurch gekennzeichnet, daß man das Carbonsäurehalogenid bzw. Carboxylat-Salz als Acylierungsreagenz oder Alkylierungsreagenz verwendet und freigesetztes $M^{n+}(Hal^{-})_n$ erneut mit Carbonsäureanhydrid umsetzt und dabei regeneriert.

12. Ausgestaltung des Verfahrens nach einem der Ansprüche 1 bis 8, dadurch gekennzeichnet, daß man das Gemisch von Carbonsäurehalogenid und Carboxylat-Salz während oder nach seiner Herstellung mit einem Alkohol unter Bildung eines Carbonsäureesters umsetzt.

## Claims

1. A process for the simultaneous preparation of carboxylic acid halides and carboxylate salts formed from carboxylate anions and 1/n cations $M^{n+}$, by reaction of compounds $M^{n+}(Hal^{-})_n$, where $M^{n+}$ is an "onium" cation and $n^+$ represents the positive charge of the cation, and where $Hal^-$ represents chloride, bromide, or iodide, with a carboxylic anhydride, or by reaction of compounds $M^{n+}(Hal^{-})_n$, where $M^{n+}$ represents the cation of a metal of the first, second or third main group or an "onium" cation, $n^+$ represents the positive charge of the cation and $Hal^-$ represents chloride or bromide, with a carboxylic anhydride of a carboxylic acid having 2 to 4 C atoms, which is substituted by 1 to 7 halogen atoms.

2. A process according to Claim 1, characterized in that the $M^{n+}(Hal^{-})_n$ is used in the form of anhydrous waste products, e.g. spent catalyst preparations, containing $M^{n+}(Hal^{-})_n$.

3. A process according to Claim 1 or 2, characterized in that carboxylic anhydrides of carboxylic acids with 2 to 4 C atoms are used.

4. A process according to one of the preceding claims, characterized in that carboxylic anhydrides of carboxylic acids with 2 to 4 C atoms which are substituted by 1 to 7 halogen atoms, preferably fluorine atoms, are used.

5. A process according to one of the preceding claims, characterized in that $M^{n+}$ represents an "onium" cation of nitrogen.

6. A process according to Claim 5, characterized in that $M^{n+}$ represents an "onium" cation of nitrogen of the formula $R^1R^2R^3R^4N^+$, in which $R^1$, $R^2$, $R^3$ and $R^4$ are, independently of each other, hydrogen, alkyl with 1 to 20 C atoms, aryl or aralkyl, or in which $R^1$ and $R^2$, or in which $R^3$ and $R^4$, or in which $R^1$, $R^2$ and $R^3$ or in which $R^1$, $R^2$, $R^3$ and $R^4$, optionally with inclusion of the nitrogen atom, form saturated or unsaturated ring systems.

7. A process according to Claim 6, characterized in that $M^{n+}$ represents ammonium, piperidinium, pyridinium or $R^{1'}R^{2'}R^{3'}R^{4'}N^+$, in which $R^{1'}$, $R^{2'}$, $R^{3'}$ and $R^{4'}$ are, independently of each other, hydrogen, alkyl with 1 to 15 C atoms or benzyl.

8. A process according to one of the preceding claims, characterized in that halide represents chloride.

9. An embodiment of the process according to one of the preceding claims, characterized in that the reaction mixture containing carboxylic acid halide and carboxylate salt is used in acylation processes.

10. A process according to one of Claims 1 to 8, characterized in that the reaction mixture is separated into carboxylic acid halide and carboxylate salt.

11. An embodiment of the process according to one of Claims 1 to 8 or 10, characterized in that the carboxylic acid halide or the carboxylate salt is used as acylating reagent or as alkylating reagent and the $M^{n+}(Hal^-)_n$ liberated is reacted anew with carboxylic anhydride and is thus regenerated.

12. An embodiment of the process according to one of Claims 1 to 8, characterized in that the mixture of carboxylic acid halide and carboxylate salt is reacted with an alcohol with the formation of a carboxylic acid ester, during or after the preparation thereof.

**Revendications**

1. Procédé de préparation simultanée d'halogénures d'acides carboxyliques et de sels de type carboxylate, formés à partir d'anions carboxylates et de 1/n cations $M^{n+}$, par réaction de composés $M^{n+}$ $(Hal^-)_n$, formule dans laquelle $M^{n+}$ est un cation "onium" et n+ indique la charge positive du cation, et $Hal^-$ représente un ion chlorure, bromure ou iodure, avec un anhydride d'acide carboxylique ou par réaction de composés $M^{n+}$ $(Hal^-)_n$, formule dans laquelle $M^{n+}$ représente le cation d'un métal du premier, du second, du troisième groupe principal ou d'un cation "onium", n+ indique la charge positive du cation et $Hal^-$ signifie un ion chlorure ou bromure, avec un anhydride d'acide carboxylique dérivant d'un acide carboxylique ayant 2 à 4 atomes de carbone, qui est substitué par 1 à 7 atome(s) d'halogène.

2. Procédé selon la revendication 1, caractérisé en ce qu'on utilise le $M^{n+}$ $(Hal^-)_n$ sous forme de produits résiduaires anhydres, par exemple de préparations de catalyseurs usées, qui contiennent $M^{n+}$ $(Hal^-)_n$.

3. Procédé selon la revendication 1 ou 2, caractérisé en ce qu'on utilise des anhydrides d'acides carboxyliques dérivant d'acides carboxyliques ayant 2 à 4 atomes de carbone.

4. Procédé selon l'une des revendications précédentes, caractérisé en ce qu'on utilise des anhydrides d'acides carboxyliques dérivant d'acides carboxyliques ayant 2 à 4 atomes de carbone, et qui sont substitués par 1 à 7 atomes d'halogène, avantageusement par des atomes de fluor.

5. Procédé selon l'une des revendications précédentes, caractérisé en ce que $M^{n+}$ représente un cation "onium" de l'azote.

6. Procédé selon la revendication 5, caractérisé en ce que $M^{n+}$ représente un cation "onium" de l'azote de formule $R^1R^2R^3R^4N^+$, dans laquelle $R^1$, $R^2$, $R^3$ et $R^4$ représentent chacun, indépendamment les uns des autres, un atome d'hydrogène, un groupe alkyle ayant 1 à 20 atomes de carbone, aryle ou aralkyle, ou bien $R^1$ et $R^2$ ou $R^3$ et $R^4$ ou $R^1$, $R^2$ et $R^3$ ou $R^1$, $R^2$,$R^3$ et $R^4$ forment, éventuellement avec inclusion de l'atome d'azote, des systèmes cycliques saturés ou insaturés.

7. Procédé selon la revendication 6, caractérisé en ce que $M^{n+}$ représente un cation ammonium, pipéridinium, pyridinium ou $R^{1'}R^{2'}R^{3'}R^{4'}N^+$, formule dans laquelle $R^{1'}$, $R^{2'}$, $R^{3'}$ et $R^{4'}$ représentent chacun, indépendamment l'un de l'autre, un hydrogène, un alkyle ayant 1 à 15 atomes de carbone ou un benzyle.

8. Procédé selon l'une des revendications précédentes, caractérisé en ce que le terme halogénure représente un chlorure.

9. Mise en oeuvre du procédé selon l'une des revendications précédentes, caractérisé en ce qu'on utilise le mélange réactionnel, contenant l'halogénure d'acide carboxylique et le sel de type carboxylate, dans un procédé d'acylation.

10. Procédé selon l'une des revendications 1 à 8, caractérisé en ce qu'on sépare le mélange réactionnel en halogénure d'acide carboxylique et en sel de type carboxylate.

11. Mise en oeuvre du procédé selon l'une des revendications 1 à 8 ou 10, caractérisé en ce qu'on utilise l'halogénure d'acide carboxylique ou le sel de type carboxylate comme agent d'acylation ou agent d'alkylation et l'on fait réagir à nouveau $M^{n+}$ $(Hal^-)_n$ ainsi libéré avec l'anhydride d'acide carboxylique et l'on effectue ainsi une régénération.

12. Mise en oeuvre du procédé selon l'une des revendications 1 à 8, caractérisé en ce qu'on fait réagir le mélange d'un halogénure d'acide carboxylique et du sel de type carboxylate, pendant ou après sa préparation, avec un alcool avec formation d'un ester d'acide carboxylique.